# EUROPÄISCHE PATENTSCHRIFT

(11) **EP 2 692 313 B1**
(45) Veröffentlichungstag und Bekanntmachung des Hinweises auf die Patenterteilung: **05.04.2017**
(21) Anmeldenummer: 13173680.3
(22) Anmeldetag: 26.06.2013
(51) Int. Cl.: A61F 2/18

(54) **Septum-Implantat**
Septum implant
Implant de septum

(30) Priorität: 03.08.2012 DE 102012107123
(43) Veröffentlichungstag der Anmeldung: 05.02.2014
(73) Patentinhaber: Heinz Kurz GmbH Medizintechnik, 72144 Dusslingen (DE)
(72) Erfinder: àWengen, Daniel Felix, 4103 Bottmingen (CH); Steinhardt, Uwe, 72145 Hirrlingen (DE)
(74) Vertreter: Kohler Schmid Möbus Patentanwälte

(56) Entgegenhaltungen:
- EP-A1- 1 475 056
- US-A1- 2007 270 899
- US-A1- 2012 078 367
- US-B1- 6 322 590

## Beschreibung

Die Erfindung betrifft ein rhinologisches Implantat zur Begradigung der Nasenscheidewand, das beiderseits des Septums der menschlichen Nase auf der jeweiligen Außenfläche des Septums in der jeweils linken und rechten Seite der Nasenhöhle befestigbar ist, wobei das Implantat vor der Implantation aus einem zunächst flachen Zuschnitt in seine spätere, implantierfähige Raumform aufgefaltet ist.

Ein solches Implantat zur Septum-Begradigung wurde beispielsweise am 26.07.2012 beschrieben auf der Internetseite http://www.bess.de/rhinologie/septumschienen/septumschiene-nach-reuter.html

Die Einpflanzung eines Implantats, d. h. eines in der Regel körperfremden Gewebsstücks oder Stoffes, in den menschlichen Körper ist in der Medizintechnik ein lange bekanntes Verfahren, das in vielen Variationen zur Behebung funktioneller Störungen verschiedener Körperteile und/oder psychischer Beeinträchtigungen vorgenommen wird.

Bekanntlich besteht der knorpelige Anteil der menschlichen Nase aus der Nasenscheidewand (=Septum), dem Dreiecksknorpel und dem Flügelknorpel. Die vorliegende Erfindung beschäftigt sich mit der Begradigung des Septums. In vielen Fällen der funktionellen, aber auch der kosmetischen Nasenchirurgie ist es das Ziel, das Septum zu begradigen, um den Luftstrom innerhalb des Nasenverlaufs zu verbessern oder schlicht die Nase kosmetisch zu verschönern. Meist geht dieser Vorgang noch mit der Veränderung anderer Strukturen einher. Derzeit werden zur Begradigung des Septums unterschiedliche Naht- oder Knorpeltransplantattechniken verwendet.

Im Folgenden sollen zunächst die Unterschiede zwischen einer Septumplastik und der Columella Strut Technik erläutert werden:
Die Septumplastik zielt ausschließlich darauf ab, das Septum zu begradigen und damit die Luftzufuhr über die Nase zu optimieren. Es handelt sich also um rein funktionelle Chirurgie. Hierzu gibt es eine Reihe von chirurgischen Techniken, die über Nähte und Transplantate zum Ziel führen sollen.

Die Begradigung eines deviierten Septums kann eine der größten Herausforderungen bei einer Rhinoplastik sein. Unterschiedliche und im Wesentlichen von der Erfahrung und dem Geschick des Operateurs abhängige Verfahren kommen hierfür in Betracht.

Disartikulationstechniken bis hin zu gezielten Knorpelinzisionen können mehr oder weniger eine Rolle spielen. In einer Sache ist sich die Fachwelt hier einig, dass eine Septumbegradigung gerade im mittleren Gewölbe eine schwierige Angelegenheit und keineswegs eine einfache Operation ist.

Im Gegensatz zur Septumbegradigung zielt die Columella Strut Technik darauf ab, die Nasenspitze aus kosmetischen Gründen heraus zu erhöhen. Dabei wird ein Steg zwischen die beiden Enden der beiden Flügelknorpel geschoben, um die Spitze der Nase zu erhöhen. Das Transplantat/ Implantat wird in eine Tasche zwischen den medialen Schenkeln der Flügelknorpel über die Spina nasalis anterior aufgestellt und zwischen den medialen Schenkeln mit durchgreifenden Nähten befestigt.

Dies ist beispielsweise beschrieben in der US 2012/0078367 A1. Hier wird von einem Implantat gesprochen, das biologisch abgebaut wird und somit nicht für den Langzeitverbleib vorgesehen ist. Die Zielregion ist das untere Drittel der Nase, hauptsächlich die Region um die Nasenspitze. Zwar ist hier auch die Rede von einer Anbindung des Implantates an das Septum, allerdings nicht zur Begradigung desselben, sondern zur Verlängerung, falls durch äußere Einwirkung oder aus kosmetischer Sicht das Septum verkürzt ist. Die gebogenen Teile des beschriebenen Implantats sind in ihrer Längenausdehnung höchstens etwa 20mm lang, d.h. sie reichen, wenn man sie auf die Basis der Spina Nasalis setzt und die beiden Schenkel der Flügelknorpel aufnimmt, gerade noch ans Ende des Septums. Letzteres wird damit also niemals begradigt. Bereits der Sitz des Implantats im implantierten Zustand wäre für Septumbegradigung völlig ungeeignet.

Ein bioabsorbierbares Columellar Strut Implantat ist aus US 2012/0078367 A1 bekannt. Auch in der WO 2008/153263 A1 werden ausschließlich Techniken beschrieben, die zum Ziel haben, die Nasenspitze anzuheben. So ist dort zwar ebenfalls vom Septum die Rede. Beschrieben wird etwa die Technik, dass das Implantat an der oberen Grenze des Septumknorpels angebracht werden muss. Allerding geschieht dies auch hier wieder lediglich zur Stabilisierung des Nasenspitzenimplantats.

Die bekannten Implantsysteme nach US 2012/0078367 A1 und nach WO 2008/153263 A1 versuchen also, das Ende des Septums zur Stabilisierung oder zur Verlängerung zu verwenden. Keines der beiden Systeme zielt jedoch darauf ab oder wäre geeignet dazu, das Septum dauerhaft zu begradigen.

Zur Begradigung des Septums hingegen sind Techniken mit PDS-Folien bekannt (etwa beschrieben in: HNO.1999 Jun;47 (6):546-50), welche zurechtgeschnitten und auf das Septum aufgenäht werden. Die hauchdünnen Folien-Implantate resorbieren nach 10 bis 25 Wochen im Wesentlichen rückstandsfrei.

Stabilere Implantate zur Septumbegradigung stellen die Septumschienen nach Reuter aus Silikon dar, wie sie auf der eingangs zitierten Internetseite der Firma Bess beschrieben sind. Diese Implantate dienen zur Schienung des Septums und zur Minimierung des Adhäsionsrisikos zwischen Septum und lateraler Nasenwand nach Rhinoplastik. Sie sind zum leichteren Einführen und Entfernen geschlitzt und weisen vorgestanzte Löcher zur Nahtbefestigung auf. Als Material wird Fluorplastik angegeben. Die Schienen müssen allerdings nach ein paar Wochen wieder aus der Nase herausgenommen werden, da Silikon als nicht langzeit-stabil gilt und potentiell zu Infektionen führt.

Der Erfindung liegt demgegenüber die Aufgabe zugrunde, mit unaufwändigen technischen Mitteln ein Implantat der gattungsbildenden Art mit den eingangs definierten Merkmalen dahingehend zu modifizieren, dass die Nachteile der Septumschienen nach Reuter vermieden werden und das Implantat nach der Operation dauerhaft möglichst eng an der Nasenscheidewand anliegt.

Diese Aufgabe wird auf überraschend einfach und kostengünstig zu realisierende Weise dadurch gelöst, dass das Implantat einen zentralen Rückenabschnitt aufweist, der flach oder nur sehr leicht von einer Horizontalebene nach oben mit einem Spreizungswinkel ω>100°, vorzugsweise ω>160° abgewinkelt oder tonnenförmig mit einem Krümmungsradius r≥0,5mm von der Horizontalebene nach oben gekrümmt ist und im implantierten Zustand die freie Unterkante des Septums umgreift, und dass zwei Seitenabschnitte des Implantats beiderseits des zentralen Rückenabschnitts vorgesehen sind, die symmetrisch zum zentralen Rückenabschnitt unter einem Winkel ϕ von jeweils etwa 90° gegen den zentralen Rückenabschnitt nach oben abgekantet verlaufen, und die im implantierten Zustand auf den beiden gegenüberliegenden Außenflächen des Septums vollflächig anliegen.

Die neue, erfindungsgemäße Grundidee ist es, das Septum einfach mit einer speziell geformten und dadurch mechanisch dauerhaft stabilen Struktur zu begradigen. Dabei ist es wichtig, dass das Implantat geometrisch so ausgebildet ist, dass der gesamte Bereich des Septums abgedeckt wird. Dies wird dadurch erreicht, dass das erfindungsgemäße Implantat die freie Unterkante des Septums umgreift. Weiter sollten beide Seiten des Implantats so perforiert sein, so dass das Implantat einfach und sicher an beiden Seiten des Septums fixiert werden kann. Die Dicke und damit einhergehende Steifigkeit des Implantats ist insofern wichtig, als dass der Septumknorpel sich langfristig der Implantatform angleichen sollte und nicht umgekehrt.

Damit wird ohne größeren Fertigungsaufwand eine besonders gute geometrische Anpassung des Implantats an die (normale) Ausgestaltung des menschlichen Septums und eine besonders gute Anlage des Implantats an den Außenflächen desselben erreicht. Insbesondere entstehen nach der Operation keine Hohlräume zwischen dem Implantat und dem Septum, weder im Bereich des zentralen Rückenabschnitts noch an den beiderseits daran anschließenden Seitenabschnitten. Vielmehr liegt das Implantat über seine gesamte dem Septum zugewandte Oberfläche eng an diesem an, was auch einen besonders guten mechanischen Halt des Implantats am Septum bewirkt.

Die beiden Seitenabschnitte des Implantats weisen jeweils einen ersten Teilabschnitt auf, der sich unmittelbar an den zentralen Rückenabschnitt anschließt und im Wesentlichen parallel zu diesem verläuft, und die beiden Seitenabschnitte weisen zu ihren freien Enden hin jeweils einen zweiten Teilabschnitt auf, der sich an den jeweiligen ersten Teilabschnitt anschließt und gegenüber diesem abgewinkelt verläuft. Durch diese einfachen Maßnahmen kann die mechanische Langzeit-Stabilität des Implantats erheblich erhöht werden. Außerdem wird der flächige feste Sitz des Implantats an den beiden Seitenflächen der Nasenscheidewand noch weiter verbessert.

Für die Form des Implantats gibt es mehrere Möglichkeiten. In einer einfachen Ausführung kann das Implantat eine winkelförmige Kontur aufweisen, insbesondere in der Ebene V-förmig gebogen sein, wobei zweckmäßigerweise die Spitze des "V" etwas abgerundet sein sollte, um Verletzungen zu vermeiden. Besonders bevorzugt weist das Implantat eine Bumerang-förmige Kontur auf.

Das Implantat kann auch trapezförmig gestaltet sein und/oder kompliziertere Strukturen mit Verzweigungen aufweisen, die nach dem Aufbiegen des Implantats in seine räumliche Endform zu einer Stabilisierung beitragen können. Bei vorteilhaften Ausführungsformen der Erfindung sind die Abkantungswinkel der freien Enden der beiden Seitenabschnitte so gestaltet, dass sich die Seitenabschnitte im implantierten Zustand in einem engen räumlichen Kontakt, insbesondere in einer beiderseits unter Spannung stehenden, vorzugsweise symmetrischen Verklemmung mit dem Septum befinden, was zu einem besonders guten und dauerhaften Sitz des Implantats beiträgt.

Bevorzugt sind Ausführungsformen des erfindungsgemäßen Implantats, bei welchen die Seitenabschnitte abgerundete Ecken aufweisen, um einerseits während der Implantation ein Hängenbleiben oder gar Aufspießen sicher zu verhindern, andererseits auch im implantierten Zustand eine Verletzungsgefahr auszuschließen.

Bei einer Klasse von vorteilhaften Ausführungsformen der Erfindung ist der Rückenabschnitt aus Stegen einer Netzstruktur aufgebaut, um dem Implantat in diesem Bereich eine erhöhte Flexibilität durch Dehnbarkeit und Stauchbarkeit zu verleihen.

Ergänzend oder alternativ können bei weiteren Ausführungsformen auch in die Seitenabschnitte Stege einer Netzstruktur eingebaut sein, was die flächenmäßige Flexibilität noch weiter erhöht.

Besonders vorteilhaft sind auch Ausführungsformen des erfindungsgemäßen Implantats, die sich dadurch auszeichnen, dass in die Seitenabschnitte ein oder mehrere Spikes eingearbeitet sind. Letztere verkrallen sich bei der Implantation im Septum-Knorpel und sorgen so für einen exzellenten und äußerst dauerhaften Halt des Implantats.

In einer weiteren sehr vorteilhaften Ausgestaltung kann das Implantat Perforationen aufweisen. Damit wird einerseits das Gewicht des Implantats verringert, andererseits der Anteil von körperfremdem Material, das durch das Implantat in den menschlichen Körper eingebracht wird, so weit wie möglich reduziert. Außerdem fördern die Perforationen das Verwachsen des Implantats mit dem Gewebe.

Die Perforationen sind vorzugsweise sowohl an den Seitenabschnitten des Implantats als auch an dem dazwischen liegenden Rückenabschnitt vorhanden und zum Beispiel als kreisrunde Löcher oder als Langlöcher ausgebildet. Weiter dienen die Perforationen einer sicheren Fixierung am Dreiecksknorpel mittels einer Naht.

Die Herstellung einer dauer-haltbaren Befestigung des Implantats durch Aufnähen am Knorpel ist allerdings in der Regel zeitintensiv und manchmal auch aufgrund der räumlichen Verhältnisse etwas kompliziert. Bei vorteilhaften Ausführungsformen der Erfindung ist daher ein im implantierten Zustand des Implantats das Septum vollständig durchquerendes, die beiden bezüglich des Septums gegenüberliegenden Seitenabschnitte miteinander verbindendes Verbindungselement, vorzugsweise ein Nietelement, vorhanden, mit welchem das Implantat über eine einfach herzustellende Perforation durch das Septum dauerhaft und sicher befestigt werden kann. Diese Art der Befestigung kann freilich auch mit den anderen, oben beschriebenen Befestigungsarten kombiniert werden, um einen besonders gesicherten Sitz des Implantats am Septum zu garantieren.

Als Material für chirurgische und orthopädische Implantate, die dauerhaft im Körper verbleiben sollen, sind Metalle und deren Legierungen prädestiniert, weil sie neben einer sehr guten Biokompatibilität hohe Dauerfestigkeit und Elastizität aufweisen. Trotz einer relativ geringen Dichte besitzen Implantate aus solchen Materialien wie Titan oder Titanverbindungen ausgezeichnete mechanische Eigenschaften mit einer langen Lebensdauer. Ebenfalls eine gute Eignung für die genannten Zwecke besitzt Edelstahl. Das erfindungsgemäße Implantat kann zwar prinzipiell auch aus einem geeigneten Kunststoff bestehen, wird aber vorzugsweise aus Metall, insbesondere aus Titan, einer Titanlegierung oder Edelstahl gefertigt sein.

Bevorzugt sind Ausführungsformen, bei welchen das Implantat aus einem Werkstoff mit Memory-Effekt und/oder superelastischen Eigenschaften, vorzugsweise aus Nitinol, gefertigt ist, so dass beispielsweise durch geeignete thermische Behandlung des Implantats optimale Federeigenschaften relativ zum Septum eingebracht werden können.

Zusätzlich zur guten Biokompatibilität des verwendeten Materials selbst kann das Implantat auch einen besonderen, körperverträglichen und/oder keimabweisenden Überzug aufweisen.

Zur Erzielung einer fein abgestimmten Form des Implantats kann dieses zweckmäßigerweise mittels Lasertechnik hergestellt sein.

Bei weiteren vorteilhaften Ausführungsformen der Erfindung schließlich ist das Implantat im Spritzguss nach dem Micro Injection Moulding (=MIM) Verfahren gefertigt, welches beispielsweise aus der WO 00/06327 A2 an sich bekannt ist. Damit kann eine äußerst preisgünstige Herstellung auch sehr großer Stückzahlen bei gleich bleibender Maßgenauigkeit erreicht werden, während die herkömmlichen Implantate in der Regel wie etwa Schmuckwaren handangefertigt werden und daher einerseits relativ teuer in der Herstellung sind, andererseits in der Maßgenauigkeit individuell variieren können.

Weitere Merkmale und Vorteile der Erfindung ergeben sich aus der nachfolgenden detaillierten Beschreibung von Ausführungsbeispielen der Erfindung anhand der Figuren der Zeichnung, die erfindungswesentliche Einzelheiten zeigt, sowie aus den Ansprüchen. Die einzelnen Merkmale können je einzeln für sich oder zu mehreren in beliebigen Kombinationen bei Varianten der Erfindung verwirklicht sein.

In der schematischen Zeichnung sind Ausführungsbeispiele der Erfindung dargestellt, welche in der nachfolgenden Beschreibung näher erläutert werden.

Es zeigen:
- Fign.1a-e: schematische perspektivische Ansichten von Ausführungsformen des erfindungsgemäßen Implantats jeweils mit dem Rückenabschnitt zwischen zwei Schmalseiten der beiden Seitenabschnitte;
- Fig. 2a: einen schematischen Schnitt durch die beiden Seitenabschnitte eines Implantats wie in Fig. 1a mit einem mit Krümmungsradius r gerundeten Rückenabschnitt;
- Fig. 2b: wie Fig. 2a, aber mit einem im Wesentlichen flachen Rückenabschnitt;
- Fig. 2c: wie Fig. 2b, aber mit einem mit Spreizungswinkel ω abgewinkelten, dachförmigen Rückenabschnitt;
- Fig. 3a: eine schematische perspektivische Ansicht einer Ausführungsform mit dem Rückenabschnitt zwischen zwei Längsseiten der beiden Seitenabschnitte im implantierten Zustand auf dem schematisch angedeuteten Septum-Knorpel, wobei an den freien Enden der zweiten Teilabschnitte der beiden Seitenabschnitte jeweils Spikes in den Knorpel eingreifen;
- Fig. 3b: wie Fig. 3a, aber ohne den Septum-Knorpel und mit zusätzlichen Spikes an den dem Rückenabschnitt abgewandten freien Kanten der beiden Seitenabschnitte;
- Fig. 3c: die Ausführungsform von Fig. 3a ohne den dort dargestellten Septum-Knorpel;
- Fig. 3d: eine vergrößerte Darstellung der Ausführungsform von Fig. 3c im Bereich der Spikes;
- Fig. 4a: eine schematische perspektivische Ansicht einer Ausführungsform mit einer den angedeuteten Septum-Knorpel nietartig durchquerendem, die beiden Seitenabschnitte im implantierten Zustand miteinander verbindendem Verbindungselement;
- Fig. 4b: einen Ausschnitt aus Fig. 4a mit Schnitt durch das Implantat und das Septum im Bereich des nietartigen Verbindungselements; und
- Fig. 4c: einen vergrößerten schematischen Schnitt durch die beiden Seitenabschnitte der Ausführungsform nach Fig. 4a mit dazwischen liegendem Septum und Verbindungselement.

Das in den Figuren der Zeichnung gezeigte rhinologische **Implantat 1a; 1b; 1c; 1d; 1e; 2a; 2b; 2c; 3a; 3b; 4a** zur Begradigung der Nasenscheidewand ist beiderseits des **Septums S** der menschlichen Nase auf der jeweiligen Außenfläche des Septums S in der jeweils linken und rechten Seite der Nasenhöhle befestigbar. Vor der Implantation wird das Implantat 1a; 1b; 1c; 1d; 1e; 2a; 2b; 2c; 3a; 3b; 4a aus einem zunächst flachen Zuschnitt in seine spätere, implantierfähige Raumform aufgefaltet.

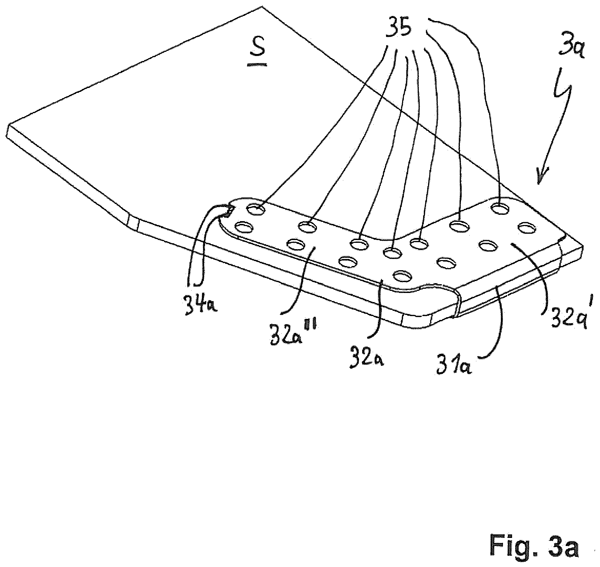

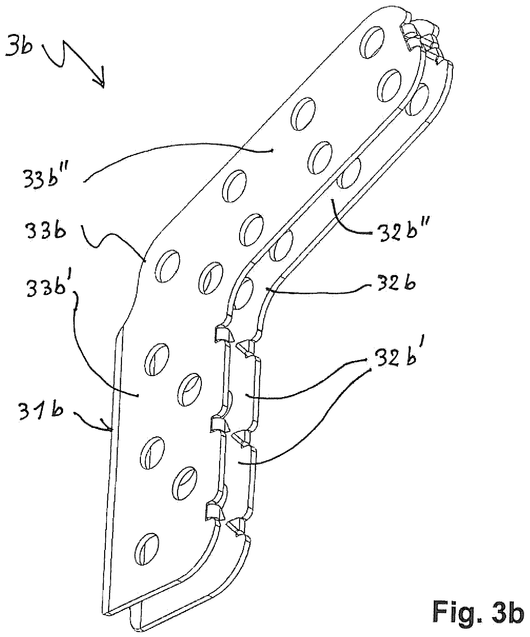

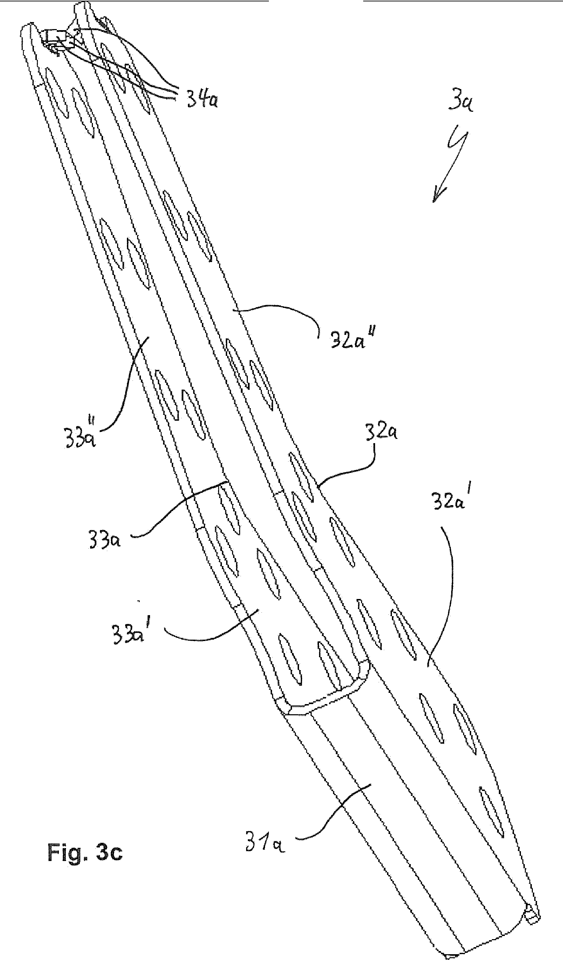

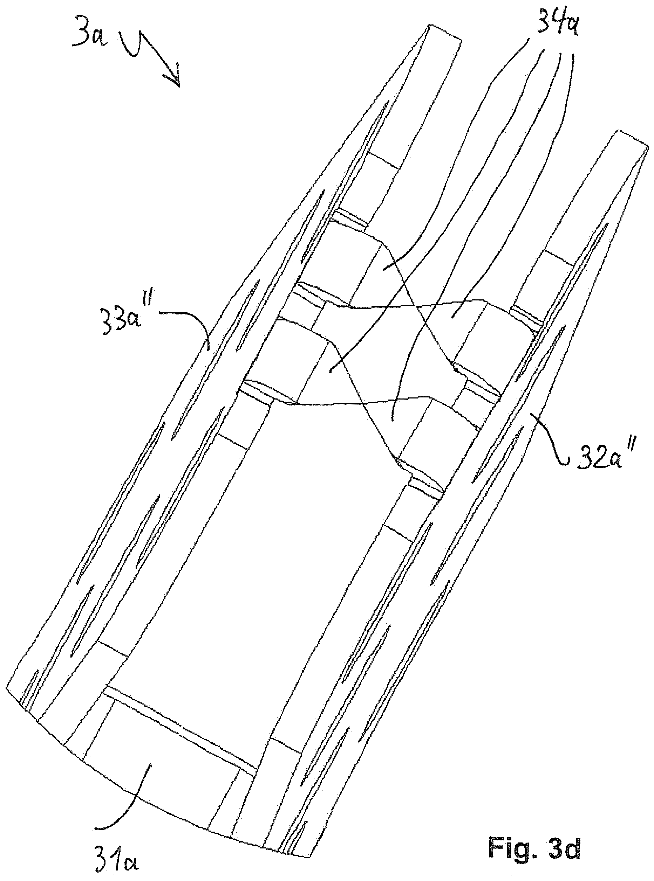

Erfindungsgemäß zeichnet sich das Implantat 1a; 1b; 1c; 1d; 1e; 2a; 2b; 2c; 3a; 3b; 4a dadurch aus, dass es einen **zentralen Rückenabschnitt 11a; 11b; 11c; 11d; 11e; 21a; 21b; 21c; 31a; 31b; 41a** aufweist, der flach oder nur sehr leicht von einer Horizontalebene nach oben mit einem Spreizungswinkel ω>100°, vorzugsweise ω>160° abgewinkelt oder tonnenförmig mit einem Krümmungsradius r≥0,5mm von der Horizontalebene nach oben gekrümmt ist und im implantierten Zustand die freie Unterkante des Septums umgreift. Zwei **Seitenabschnitte 12a,13a; 12b,13b; 12c,13c; 12d,13d; 12e,13e; 22a,23a; 22b,23b; 22c,23c; 32a,33a; 32b,33b; 42a,43a** sind beiderseits des zentralen Rückenabschnitts 11a; 11b; 11c; 11d; 11e; 21a; 21b; 21c; 31a; 31b; 41a vorgesehen, die symmetrisch zum zentralen Rückenabschnitt 11a; 11b; 11c; 11d; 11e; 21a; 21b; 21c; 31a; 31b; 41a unter einem Winkel ϕ von jeweils etwa 90° gegen den zentralen Rückenabschnitt 11a; 11b; 11c; 11d; 11e; 21a; 21b; 21c; 31a; 31b; 41a nach oben abgekantet verlaufen, und die im implantierten Zustand auf den beiden gegenüberliegenden Außenflächen des Septums S vollflächig anliegen, wie besonders gut in der Darstellung von Fig. 3a zu erkennen ist.

Bei sämtlichen in der Zeichnung dargestellten Ausführungsformen sind die Ecken der Seitenabschnitte 12a,13a; 12b,13b; 12c,13c; 12d,13d; 12e,13e; 22a,23a; 22b,23b; 22c,23c; 32a,33a; 32b,33b; 42a,43a abgerundet.

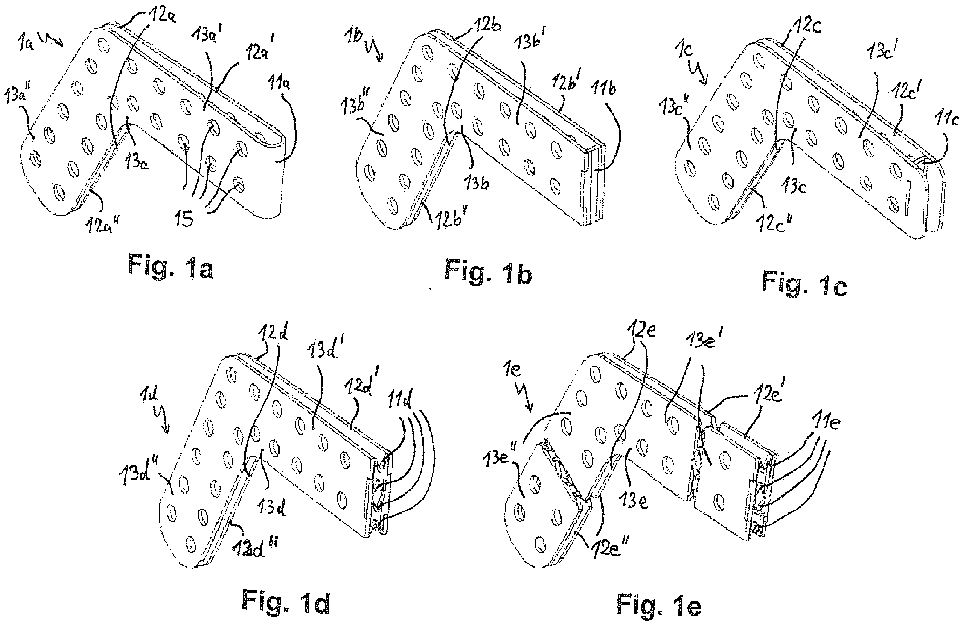

Die Figuren 1a bis 1e zeigen in schematischen perspektivischen Ansichten fünf Ausführungsformen 1a; 1b; 1c; 1d; 1e eines erfindungsgemäßen Implantats. Dieses ist jeweils aus einem ebenen Streifen in eine dachartige Raumform gebogen, was aber in der Zeichnung nicht eigens dargestellt ist.

Mit den in den Figuren 3a bis 3d dargestellten Ausführungsformen 3a; 3b der Erfindung haben die Implantate 1a; 1b; 1c; 1d; 1e gemeinsam, dass die beiden Seitenabschnitte 12a,13a; 12b,13b; 12c,13c; 12d,13d; 12e,13e; 32a,33a; 32b,33b jeweils einen **ersten Teilabschnitt 12a',13a'; 12b',13b'; 12c',13c'; 12d',13d'; 12e',13e';32a',33a'; 32b',33b'** aufweisen, der sich unmittelbar an den zentralen Rückenabschnitt 11a; 11b; 11c; 11d; 11e; 31a; 31b anschließt und im Wesentlichen parallel zu diesem verläuft, und dass die beiden Seitenabschnitte 12a,13a; 12b,13b; 12c,13c; 12d,13d; 12e,13e; 32a,33a; 32b,33b zu ihren freien Enden hin jeweils einen **zweiten Teilabschnitt 12a",13a"; 12b",13b"; 12c",13c"; 12d",13d"; 12e",13e";32a",33a"; 32b",33b"** aufweisen, der sich an den jeweiligen ersten Teilabschnitt 12a',13a'; 12b',13b'; 12c',13c'; 12d',13d'; 12e',13e';32a',33a'; 32b',33b' anschließt und gegenüber diesem abgewinkelt verläuft. Insbesondere kann das Implantat 1a; 1b; 1c; 1d; 1e; 3a; 3b eine winkelförmige Kontur, vorzugsweise eine V-förmige oder Bumerang-förmige Kontur aufweisen. Dadurch kann die mechanische Langzeit-Stabilität des Implantats erheblich erhöht und der flächige feste Sitz an den beiden Seitenflächen der Nasenscheidewand noch weiter verbessert werden.

Bei den in den Figuren 1d und 1e gezeigten Ausführungsformen 1d; 1e ist der Rückenabschnitt 11d; 11e aus Stegen einer Netzstruktur aufgebaut. Auch in die Seitenabschnitte 12e,13e können Stege einer Netzstruktur eingebaut sein, was jeweils die Flexibilität des Implantats erheblich erhöht und eine gewisse Variabilität bei der Anpassung auf individuell beim Patienten vorgefundene geometrische Verhältnisse ermöglicht.

Wie auch schon in den Figuren 1a bis 1e erkennbar, muss der Rückenabschnitt 11a; 11b; 11c; 11d; 11e der erfindungsgemäßen Implantate 1a; 1b; 1c; 1d; 1e nicht notwendig völlig flach gestaltet sein. Er kann bei Ausführungsformen auch eine sehr leichte Abwinkelung oder eine ganz geringe Krümmung aufweisen, ohne dass dabei die Vorteile der Erfindung insgesamt verloren gehen. Im Gegenteil ist ja bei manchen Menschen die freie Unterkante des Septums S ebenfalls nicht völlig flach, sondern ganz leicht gewölbt, so dass mit solchen Ausführungsformen eine besonders gute geometrische Anpassung des Implantats an die individuellen Gegebenheiten des Patienten vorgenommen werden kann.

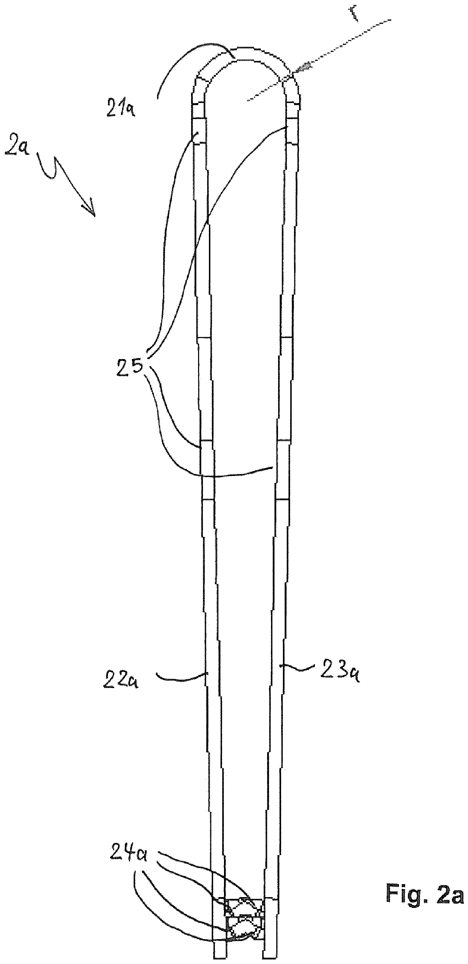

In Fig. 2a ist daher eine Ausführungsform der Erfindung dargestellt, bei der das Implantat 2a eine leichte tonnenförmige Krümmung mit einem relativ großen Krümmungsradius r≥0,5mm aufweist und damit im implantierten Zustand die freie Unterkante des Septums umgreift.

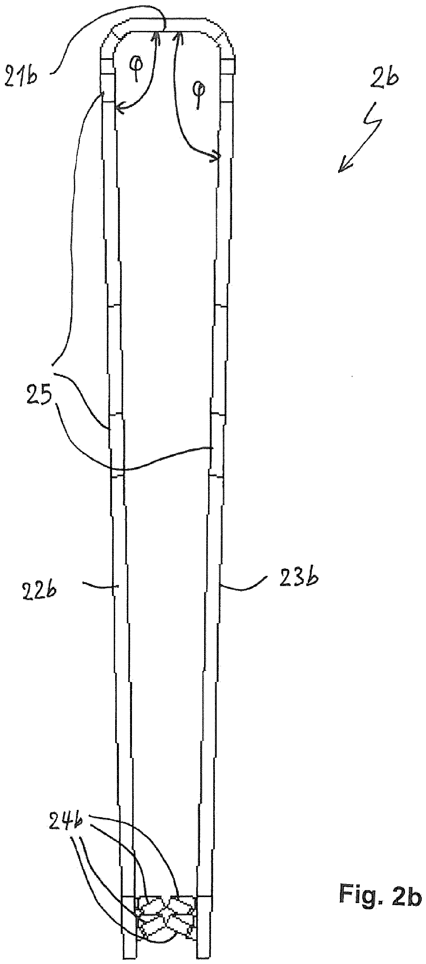

Fig. 2b zeigt dagegen eine Ausführungsform, bei der das Implantat 2b im implantierten Zustand flach an der freien Unterkante des Septums anliegt.

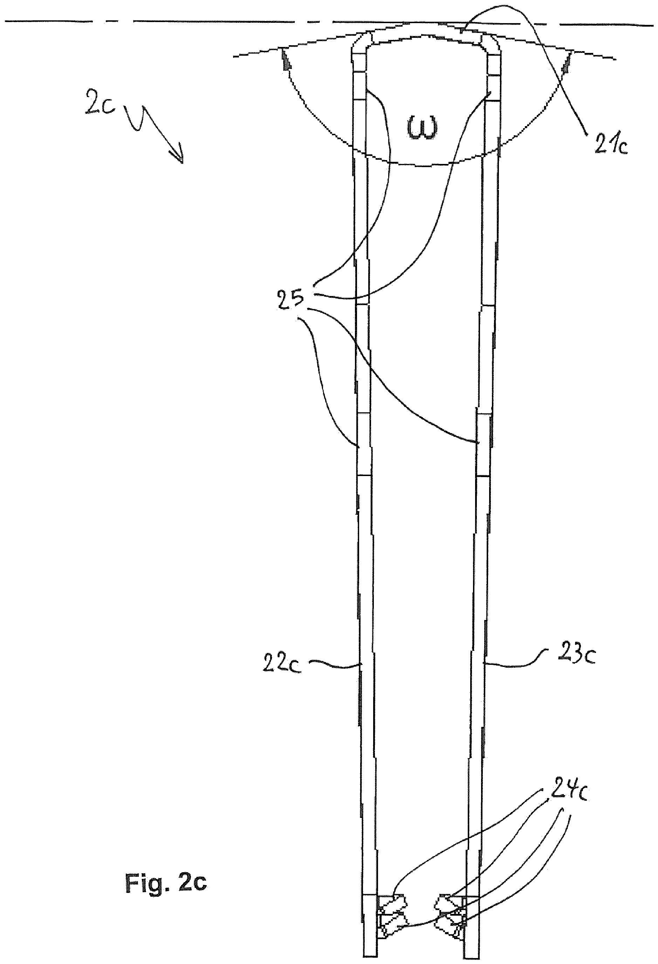

In Fig. 2c schließlich ist ein Implantat 2c dargestellt, welches von einer - imaginären - Horizontalebene mit einem Spreizungswinkel ω>100°, vorzugsweise ω>160° abgewinkelt ist und im implantierten Zustand die freie Unterkante des Septums dachförmig umgreift.

Je nach aktuell vorgefundener Ausbildung des Septums beim jeweiligen Patienten kann individuell eine optimal angepasste Geometrie des Implantats ausgewählt werden.

Wie in den Figuren 2a bis 2c, 3a bis 3d sowie 4a gezeigt, können in die Seitenabschnitte 22a,23a; 22b,23b; 22c,23c; 32a,33a; 32b,33b; 42a,43a **Spikes 24a; 24b; 24c; 34a; 44** eingearbeitet sein, die im implantierten Zustand des Implantats 2a; 2b; 2c; 3a; 3b; 4a mit dem Septum-Knorpel verkrallen und so einen besonders sicheren Halt verleihen.

Das erfindungsgemäße Implantat 1a; 1b; 1c; 1d; 1e; 2a; 2b; 2c; 3a; 3b; 4a weist bei den in der Zeichnung gezeigten Ausführungsformen jeweils **Perforationen 15; 25; 35; 45** auf. Letztere können sowohl an den Seitenabschnitten 12a,13a; 12b,13b; 12c,13c; 12d,13d; 12e,13e; 22a,23a; 22b,23b; 22c,23c; 32a,33a; 32b, 33b; 42a,43a vorgesehen sein als auch an dem dazwischen liegenden zentralen Rückenabschnitt des Implantats - was aber in der Zeichnung nicht eigens dargestellt ist.

Die Perforationen 15; 25; 35; 45 können - wie in den Figuren der Zeichnung gezeigt - als kreisrunde Löcher oder auch als Langlöcher ausgebildet sein. Sie helfen, einerseits das Gewicht des Implantats zu verringern und andererseits den Anteil des körperfremden Materials im Körper eines Patienten so weit wie möglich zu reduzieren. Außerdem fördern die Perforationen 15; 25; 35; 45 das Verwachsen des Implantats mit dem Gewebe.

Das Implantat 1a; 1b; 1c; 1d; 1e; 2a; 2b; 2c; 3a; 3b wird operativ durch eine sogenannte offene Rhinoplastik in die Nase eingebracht und auf dem Knorpel des Septums S mittels einer Naht befestigt. Hierbei werden mehrere Einzelnähte durch die Perforationen 15; 25; 35; 45 und das Septum S angelegt und fixiert.

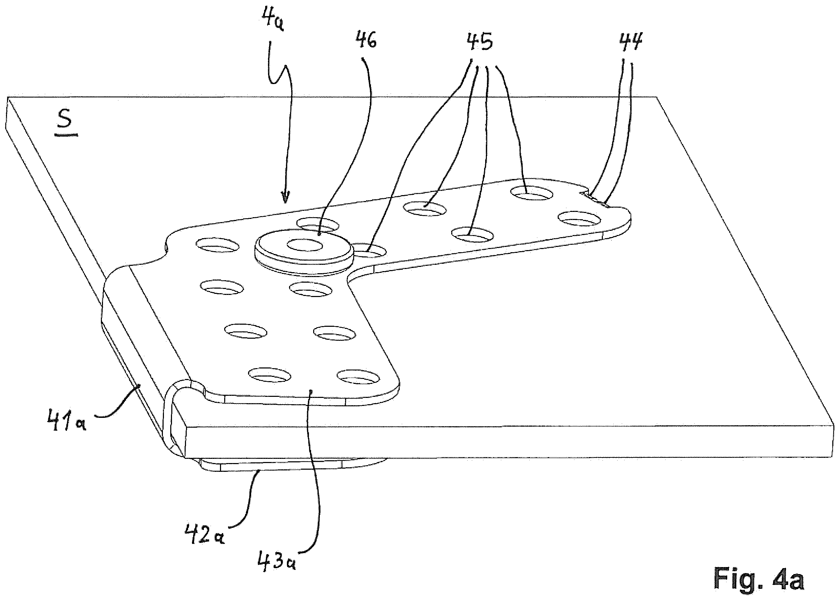

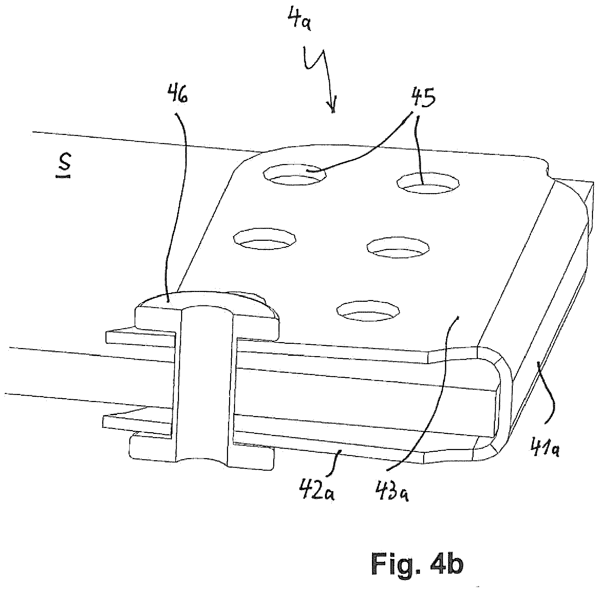

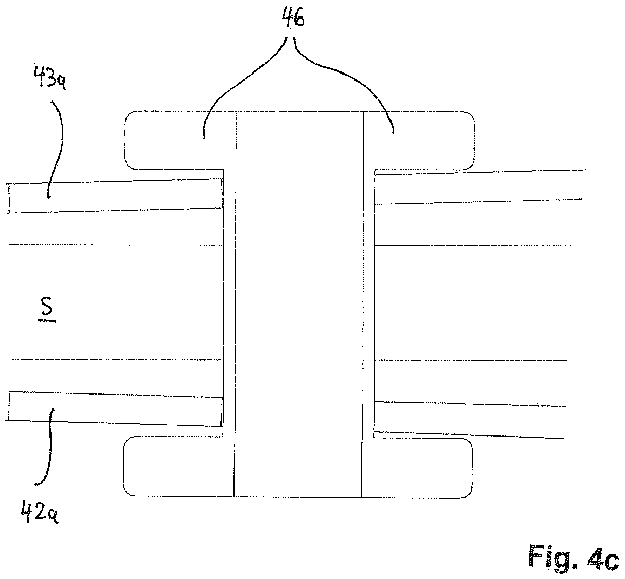

Alternativ (oder ergänzend) kann zur Befestigung des Implantats 4a - wie in den Figuren 4a bis 4c schematisch dargestellt - auch ein im implantierten Zustand des Implantats 4a das Septum S vollständig durchquerendes, die beiden bezüglich des Septums S gegenüber liegenden Seitenabschnitte 42a,43a miteinander verbindendes **Verbindungselement 46** vorgesehen sein, das vorzugsweise als Nietelement aus Metall, insbesondere aus einer Titan-Legierung, ausgebildet ist und dem Implantat 4a einen dauerhaften auf dem Septum S verleiht.

Das erfindungsgemäße Implantat 1a; 1b; 1c; 1d; 1e; 2a; 2b; 2c; 3a; 3b; 4a kann ganz oder teilweise aus Metall, insbesondere aus Titan, aus einer Titanlegierung oder aus Edelstahl und/oder aus einem Werkstoff mit Memory-Effekt und/oder superelastischen Eigenschaften, vorzugsweise aus Nitinol, gefertigt sein. Vorzugsweise wird es einen körperverträglichen Überzug aufweisen.

Das Implantat 1a; 1b; 1c; 1d; 1e; 2a; 2b; 2c; 3a; 3b; 4a kann beispielsweise mittels Lasertechnik oder auch im Spritzguss nach dem Micro Injection Moulding (=MIM) Verfahren gefertigt werden.

## Patentansprüche

1. Rhinologisches Implantat (1a; 1b; 1c; 1d; 1e; 2a; 2b; 2c; 3a; 3b; 4a) zur Begradigung der Nasenscheidewand, das beiderseits des Septums (S) der menschlichen Nase auf der jeweiligen Außenfläche des Septums (S) in der jeweils linken und rechten Seite der Nasenhöhle befestigbar ist, wobei das Implantat (1a; 1b; 1c; 1d; 1e; 2a; 2b; 2c; 3a; 3b; 4a) einen zentralen Rückenabschnitt (11a; 11b; 11c; 11d; 11e; 21a; 21b; 21c; 31a; 31b; 41a) aufweist, der flach oder nur sehr leicht von einer Horizontalebene nach oben mit einem Spreizungswinkel ω>100° abgewinkelt oder tonnenförmig mit einem Krümmungsradius r≥0,5mm von der Horizontalebene nach oben gekrümmt ist und im implantierten Zustand die freie Unterkante des Septums umgreift und wobei zwei Seitenabschnitte (12a,13a; 12b,13b; 12c,13c; 12d,13d; 12e,13e; 22a,23a; 22b,23b; 22c,23c; 32a,33a; 32b,33b; 42a,43a) des Implantats (1a; 1b; 1c; 1d; 1e; 2a; 2b; 2c; 3a; 3b; 4a) beiderseits des zentralen Rückenabschnitts (11a; 11b; 11c; 11d; 11e; 21a; 21b; 21c; 31a; 31b; 41a) vorgesehen sind, die symmetrisch zum zentralen Rückenabschnitt (11a; 11b; 11c; 11d; 11e; 21a; 21b; 21c; 31a; 31b; 41a) unter einem Winkel ϕ von jeweils etwa 90° gegen den zentralen Rückenabschnitt (11a; 11b; 11c; 11d; 11e; 21a; 21b; 21c; 31a; 31b; 41a) nach oben abgekantet verlaufen, und die im implantierten Zustand auf den beiden gegenüberliegenden Außenflächen des Septums (S) vollflächig anliegen,
**dadurch gekennzeichnet,**
**dass** das Implantat (1a; 1b; 1c; 1d; 1e; 2a; 2b; 2c; 3a; 3b; 4a) vor der Implantation aus einem zunächst flachen Zuschnitt in seine spätere, implantierfähige Raumform aufgefaltet ist, ,
und **dass** die beiden Seitenabschnitte (12a,13a; 12b,13b; 12c,13c; 12d,13d; 12e,13e; 32a,33a; 32b,33b) des Implantats (1a; 1b; 1c; 1d; 1e; 3a; 3b) jeweils einen ersten Teilabschnitt (12a',13a'; 12b',13b'; 12c',13c'; 12d',13d'; 12e',13e';32a',33a'; 32b',33b') aufweisen, der sich unmittelbar an den zentralen Rückenabschnitt (11a; 11b; 11c; 11d; 11e; 31a; 31b) anschließt und im Wesentlichen parallel zu diesem verläuft, und dass die beiden Seitenabschnitte (12a,13a; 12b,13b; 12c,13c; 12d,13d; 12e,13e; 32a,33a; 32b,33b) zu ihren freien Enden hin jeweils einen zweiten Teilabschnitt (12a",13a"; 12b",13b"; 12c",13c"; 12d",13d"; 12e",13e";32a",33a"; 32b",33b") aufweisen, der sich an den jeweiligen ersten Teilabschnitt (12a',13a'; 12b',13b'; 12c',13c'; 12d',13d'; 12e',13e';32a',33a'; 32b',33b') anschließt und gegenüber diesem abgewinkelt verläuft.

2. Implantat nach Anspruch 1, **dadurch gekennzeichnet, dass** für den Spreizungswinkel gilt ω>160°.

3. Implantat nach Anspruch 1 oder 2, **dadurch gekennzeichnet, dass** das Implantat (1a; 1b; 1c; 1d; 1e; 3a; 3b) eine winkelförmige Kontur, insbesondere eine V-förmige oder Bumerang-förmige Kontur aufweist.

4. Implantat nach einem der vorhergehenden Ansprüche, **dadurch gekennzeichnet, dass** die Ecken der Seitenabschnitte (12a,13a; 12b,13b; 12c,13c; 12d,13d; 12e,13e; 22a,23a; 22b,23b; 22c,23c; 32a,33a; 32b,33b; 42a,43a) abgerundet sind.

5. Implantat nach einem der vorhergehenden Ansprüche, **dadurch gekennzeichnet, dass** der Rückenabschnitt (11d; 11e) aus Stegen einer Netzstruktur aufgebaut ist.

6. Implantat nach einem der vorhergehenden Ansprüche, **dadurch gekennzeichnet, dass** in die Seitenabschnitte (12e,13e) Stege einer Netzstruktur eingebaut sind.

7. Implantat nach einem der vorhergehenden Ansprüche, **dadurch gekennzeichnet, dass** in die Seitenabschnitte (22a,23a; 22b,23b; 22c,23c; 32a,33a; 32b,33b; 42a,43a) ein oder mehrere Spikes (24a; 24b; 24c; 34a; 44) eingearbeitet sind.

8. Implantat nach einem der vorhergehenden Ansprüche, **dadurch gekennzeichnet, dass** das Implantat (1a; 1b; 1c; 1d; 1e; 2a; 2b; 2c; 3a; 3b; 4a) Perforationen (15; 25; 35; 45) aufweist, die vorzugsweise als kreisrunde Löcher oder als Langlöcher ausgebildet sind.

9. Implantat nach Anspruch 8, **dadurch gekennzeichnet, dass** die Perforationen sowohl an den Seitenabschnitten als auch an dem dazwischen liegenden Rückenabschnitt des Implantats vorgesehen sind.

10. Implantat nach einem der vorhergehenden Ansprüche, **dadurch gekennzeichnet, dass** ein im implantierten Zustand des Implantats (4a) das Septum (S) vollständig durchquerendes, die beiden bezüglich des Septums (S) gegenüberliegenden Seitenabschnitte (42a,43a) miteinander verbindendes Verbindungselement (46), vorzugsweise ein Nietelement, vorgesehen ist.

11. Implantat nach einem der vorhergehenden Ansprüche, **dadurch gekennzeichnet, dass** das Implantat (1a; 1b; 1c; 1d; 1e; 2a; 2b; 2c; 3a; 3b; 4a) ganz oder teilweise aus Metall, insbesondere aus Titan, aus einer Titanlegierung oder aus Edelstahl gefertigt ist.

12. Implantat nach einem der vorhergehenden Ansprüche, **dadurch gekennzeichnet, dass** das Implantat (1a; 1b; 1c; 1d; 1e; 2a; 2b; 2c; 3a; 3b; 4a) ganz oder teilweise aus einem Werkstoff mit Memory-Effekt und/oder superelastischen Eigenschaften, vorzugsweise aus Nitinol, gefertigt ist.

13. Implantat nach einem der vorhergehenden Ansprüche, **dadurch gekennzeichnet, dass** das Implantat (1a; 1b; 1c; 1d; 1e; 2a; 2b; 2c; 3a; 3b; 4a) einen körperverträglichen Überzug aufweist.

14. Implantat nach einem der Ansprüche 1 bis 13, **dadurch gekennzeichnet, dass** das Implantat (1a; 1b; 1c; 1d; 1e; 2a; 2b; 2c; 3a; 3b; 4a) mittels Lasertechnik hergestellt ist.

15. Implantat nach einem der Ansprüche 1 bis 13, **dadurch gekennzeichnet, dass** das Implantat (1a; 1b; 1c; 1d; 1e; 2a; 2b; 2c; 3a; 3b; 4a) im Spritzguss nach dem Micro Injection Moulding (=MIM) Verfahren gefertigt ist.

## Claims

1. Rhinological implant (1a; 1b; 1c; 1d; 1e; 2a; 2b; 2c; 3a; 3b; 4a) for straightening the nasal septum, which may be fixed on both sides of the septum (S) of the human nose on the relevant outside surface of the septum (S) in the left and right side of the nasal cavity, in which the implant (1a; 1b; 1c; 1d; 1e; 2a; 2b; 2c; 3a; 3b; 4a) has a central back section (11a; 11b; 11c; 11d; 11e; 21a; 21b; 21c; 31a; 31b; 41a), which is flat or only very slightly angled upwards from a horizontal plane with a spreading angle ω > 100° or barrel shaped curved upwards from the horizontal plane with a bending radius r ≥ 0.5 mm and surrounds the free bottom edge of the septum in the implanted state and in which two side sections (12a, 13a; 12b, 13b; 12c, 13c; 12d, 13d; 12e, 13e; 22a, 23a; 22b, 23b; 22c, 23c; 32a, 33a; 32b, 33b; 42a, 43a) of the implant (1a; 1b; 1c; 1d; 1e; 2a; 2b; 2c; 3a; 3b; 4a) are provided on both sides of the central back section (11a; 11b; 11c; 11d; 11e; 21a; 21b; 21c; 31a; 31b; 41a), which run symmetrically to the central back section (11a; 11b; 11c; 11d; 11e; 21a; 21b; 21c; 31a; 31b; 41a) bent upwards at an angle ϕ of about 90° to the central back section (11a; 11b; 11c; 11d; 11e; 21a; 21b; 21c; 31a; 31b; 41a), and which lie all over on both opposite outer surfaces of the septum (S) in the implanted state, **characterised in that**
before implantation the implant (1a; 1b; 1c; 1d; 1e; 2a; 2b; 2c; 3a; 3b; 4a) is unfolded from a first flat section into its later shape that may be implanted, and that both side sections (12a, 13a; 12b, 13b; 12c, 13c; 12d, 13d; 12e, 13e; 32a, 33a; 32b, 33b) of the implant (1a; 1b; 1c; 1d; 1e; 3a; 3b) have a first part section (12a', 13a'; 12b', 13b'; 12c', 13c'; 12d', 13d'; 12e', 13e'; 32a', 33a'; 32b', 33b'), which connects directly to the central back section (11a; 11b; 11c; 11d; 11e; 31a; 31b) and runs essentially parallel to it, and that both side sections (12a, 13a; 12b, 13b; 12c, 13c; 12d, 13d; 12e, 13e; 32a, 33a; 32b, 33b) have a second part section (12a", 13a"; 12b", 13b"; 12c", 13c"; 12d", 13d"; 12e", 13e"; 32a", 33a"; 32b", 33b") at their free ends, which connects to the relevant first part section (12a', 13a'; 12b', 13b'; 12c', 13c'; 12d', 13d'; 12e', 13e'; 32a', 33a'; 32b', 33b') and runs at an angle to it.

2. Implant according to claim 1, **characterised in that** ω > 160° applies for the spreading angle.

3. Implant according to claim 1 or 2, **characterised in that** the implant (1a; 1b; 1c; 1d; 1e; 3a; 3b) has an angular contour, particularly a V-shaped or boomerang-shaped contour.

4. Implant according to one of the previous claims, **characterised in that** the corners of the side sections (12a, 13a; 12b, 13b; 12c, 13c; 12d, 13d; 12e, 13e; 22a, 23a; 22b, 23b; 22c, 23c; 32a, 33a; 32b, 33b; 42a, 43a) are rounded off.

5. Implant according to one of the previous claims, **characterised in that** the back section (11d; 11e) is constructed from the links of a network structure.

6. Implant according to one of the previous claims, **characterised in that** the links of a network structure are fitted into the side sections (12e, 13e).

7. Implant according to one of the previous claims, **characterised in that** one or several spikes (24a; 24b; 24c; 34a; 44) are incorporated into the side sections (22a, 23a; 22b, 23b; 22c, 23c; 32a, 33a; 32b, 33b; 42a, 43a).

8. Implant according to one of the previous claims, **characterised in that** the implant (1a; 1b; 1c; 1d; 1e; 2a; 2b; 2c; 3a; 3b; 4a) has perforations (15; 25; 35; 45), which are made preferably as circular holes or as oblong holes.

9. Implant according to claim 8, **characterised in that** the perforations are provided both on the side sections and on the back section of the implant lying between them.

10. Implant according to one of the previous claims, **characterised in that** a connecting element (46), preferably a rivet element, which crosses through the septum (S) completely, connecting both opposite side sections (42a, 43a) with reference to the septum (S) with each other, is provided in the implanted state of the implant (4a).

11. Implant according to one of the previous claims, **characterised in that** the implant (1a; 1b; 1c; 1d; 1e; 2a; 2b; 2c; 3a; 3b; 4a) is made completely or partly from metal, particularly titanium, a titanium alloy or a high-grade steel.

12. Implant according to one of the previous claims, **characterised in that** the implant (1a; 1b; 1c; 1d; 1e; 2a; 2b; 2c; 3a; 3b; 4a) is made completely or partly from a material with memory effect and/or superelastic properties, preferably nitinol.

13. Implant according to one of the previous claims, **characterised in that** the implant (1a; 1b; 1c; 1d; 1e; 2a; 2b; 2c; 3a; 3b; 4a) has a biocompatible coating.

14. Implant according to one of claims 1 to 13, **characterised in that** the implant (1a; 1b; 1c; 1d; 1e; 2a; 2b; 2c; 3a; 3b; 4a) is produced by means of laser technology.

15. Implant according to one of claims 1 to 13, **characterised in that** the implant (1a; 1b; 1c; 1d; 1e; 2a; 2b; 2c; 3a; 3b; 4a) is produced by injection moulding according to the Micro Injection Moulding (=MIM) process.

## Revendications

1. Implant rhinologique (1a ; 1b ; 1c ; 1d ; 1e ; 2a ; 2b ; 2c ; 3a ; 3b ; 4a) pour la rectification du septum nasal, qui est susceptible d'être fixé sur les deux côtés du septum (S) du nez humain sur la surface extérieure respective du septum (S) du côté droit et du côté gauche respectif de la cavité nasale, ledit implant (1a ; 1b ; 1c ; 1d ; 1e ; 2a ; 2b ; 2c ; 3a ; 3b ; 4a) comprenant une portion dorsale centrale (11a ; 11b ; 11c ; 11d ; 11^{e} ; 21a ; 21b ; 21c ; 31a ; 31 b ; 41a), qui forme un angle plat ou seulement très légèrement en angle depuis un plan horizontal vers le haut avec un angle d'écartement ω > 100°, ou est incurvé vers le haut depuis le plan horizontal en forme bombée avec un rayon de courbure r ≥ 0,5 mm et enserre dans l'état implanté l'arête inférieure libre du septum, et dans lequel deux portions latérales (12a, 13a ; 12b, 13b ; 12c, 13c ; 12d, 13d ; 12e, 13e ; 22a, 23a ; 22b, 23b ; 22c, 23c ; 32a, 33a ; 32b, 33b ; 42a, 43a) de l'implant (1a ; 1b ; 1c ; 1d ; 1e ; 2a ; 2b ; 2c ; 3a ; 3b ; 4a) sont prévues sur les deux côtés de la portion dorsale centrale (11a ; 11b ; 11c ; 11d ; 11e ; 21a ; 21b ; 21c ; 31a ; 31b ; 41a), qui s'étendent en équerre vers le haut et symétriquement par rapport à la portion dorsale centrale (11a ; 11b ; 11c ; 11d ; 11e ; 21a ; 21b ; 21c ; 31 a ; 31b ; 41a) sous un angle ϕ respectivement environ égal à 90° par rapport à la portion dorsale centrale (11a ; 11b ; 11c ; 11d ; 11e ; 21a ; 21b ; 21c ; 31a ; 31b ; 41a), et qui s'appliquent dans l'état implanté sur toute la surface des deux faces extérieures opposées du septum (S),
**caractérisé en ce que**
l'implant (1a ; 1b ; 1c ; 1d ; 1e ; 2a ; 2b ; 2c ; 3a ; 3b ; 4a) est tout d'abord replié, avant l'implantation, à partir d'un flan tout d'abord plat pour lui donner sa forme spatiale ultérieure susceptible d'être implanté, et
**en ce que** les deux portions latérales (12a, 13a ; 12b, 13b ; 12c, 13c ; 12d, 13d ; 12e, 13e ; 32a, 33a ; 32b, 33b) de l'implant (1a ; 1b ; 1c ; 1d ; 1e ; 3a ; 3b) comportent chacune une première portion partielle (12a', 13a' ; 12b', 13b' ; 12c', 13c' ; 12d', 13d' ; 12e', 13e' ; 32a', 33a' ; 32b', 33b'), qui se raccorde directement à sa portion dorsale centrale (11a ; 11 b ; 11c ; 11d ; 11e ; 31a ; 31b) et s'étend sensiblement parallèlement à celle-ci, et
**en ce que** les deux portions latérales (12a, 13a ; 12b, 13b ; 12c, 13c ; 12d, 13d ; 12e, 13e ; 32a, 33a ; 32b, 33b) comprennent, en direction de leurs extrémités libres, respectivement une seconde portion partielle (12a", 13a" ; 12b", 13b" ; 12c", 13c" ; 12d", 13d" ; 12e", 13e" ; 32a", 33a" ; 32b", 33b"), qui se raccorde à la première portion partielle respective (12a', 13a' ; 12b', 13b' ; 12c', 13c' ; 12d', 13d' ; 12e', 13e' ; 32a', 33a' ; 32b', 33b') et s'étend en formant un angle par rapport à celle-ci.

2. Implant selon la revendication 1, **caractérisé en ce que** l'angle d'écartement est tel que w > 160°.

3. Implant selon la revendication 1 ou 2, **caractérisé en ce que** l'implant (1a ; 1b ; 1c ; 1d ; 1e ; 3a ; 3b) présente un contour de forme angulaire, en particulier un contour en forme de V ou un contour en forme de boomerang.

4. Implant selon l'une des revendications précédentes, **caractérisé en ce que** les coins des portions latérales (12a, 13a ; 12b, 13b ; 12c, 13c ; 12d, 13d ; 12e, 13e ; 22a, 23a ; 22b, 23b ; 22c, 23c ; 32a, 33a ; 32b, 33b ; 42a, 43a) sont arrondis.

5. Implant selon l'une des revendications précédentes, **caractérisé en ce que** la portion dorsale (11d ; 11e) est réalisée à partir des barrettes d'une structure en réseau.

6. Implant selon l'une des revendications précédentes, **caractérisé en ce que** les barrettes d'une structure en réseau sont intégrées dans les portions latérales (12e, 13e).

7. Implant selon l'une des revendications précédentes, **caractérisé en ce qu'**une ou plusieurs pointes (24a ; 24b ; 24c ; 34a ; 44) sont intégrées dans les portions latérales (22a, 23a ; 22b, 23b ; 22c, 23c ; 32a, 33a ; 32b, 33b ; 42a, 43a).

8. Implant selon l'une des revendications précédentes, **caractérisé en ce que** l'implant (1a ; 1b ; 1c ; 1d ; 1e ; 2a ; 2b ; 2c ; 3a ; 3b ; 4a) comporte des perforations (15 ; 25 ; 35 ; 45), qui sont de préférence réalisées comme des trous circulaires ronds ou comme des trous oblongs.

9. Implant selon la revendication 8, **caractérisé en ce que** les perforations sont prévues aussi bien sur les portions latérales que sur la portion dorsale de l'implant disposée entre celles-ci.

10. Implant selon l'une des revendications précédentes, **caractérisé en ce qu'**il est prévu un élément de liaison (46), de préférence un élément en rivet, qui traverse entièrement le septum (S) dans l'état implanté de l'implant (4a) et qui relie l'une à l'autre les deux portions latérales (42a, 43a) opposées par rapport au septum (S).

11. Implant selon l'une des revendications précédentes, **caractérisé en ce que** l'implant (1a ; 1b ; 1c ; 1d ; 1e ; 2a ; 2b ; 2c ; 3a ; 3b ; 4a) est réalisé en totalité ou en partie en métal, en particulier en titane, en un alliage de titane ou en acier inoxydable.

12. Implant selon l'une des revendications précédentes, **caractérisé en ce que** l'implant (1a ; 1b ; 1c ; 1d ; 1e ; 2a ; 2b ; 2c ; 3a ; 3b ; 4a) est réalisé en totalité ou en partie en un matériau à effet mémoire et/ou avec des propriétés superélastiques, de préférence en nitinol.

13. Implant selon l'une des revendications précédentes, **caractérisé en ce que** l'implant (1a ; 1b ; 1c ; 1d ; 1e ; 2a ; 2b ; 2c ; 3a ; 3b ; 4a) comporte un revêtement compatible avec le corps.

14. Implant selon l'une des revendications 1 à 13, **caractérisé en ce que** l'implant (1a ; 1b ; 1c ; 1d ; 1e ; 2a ; 2b ; 2c ; 3a ; 3b ; 4a) est réalisé au moyen de techniques à laser.

15. Implant selon l'une des revendications 1 à 13, **caractérisé en ce que** l'implant (1a ; 1b ; 1c ; 1d ; 1e ; 2a ; 2b ; 2c ; 3a; 3b ; 4a) est réalisé par moulage par injection selon la procédure dite "MIM (Micro Injection Moulding ou "moulage par micro-injection").
